# EUROPEAN PATENT APPLICATION

(11) **EP 4 621 410 A1**
(43) Date of publication of application: **24.09.2025**
(21) Application number: 23899483.4
(22) Date of filing: 24.08.2023
(51) Int. Cl.: G01N 33/68

(54) **USE OF SERUM SOLUBLE ST2 IN PREPARATION FOR PRODUCT FOR PREDICTING PROGNOSIS OF PATIENT SUFFERING FROM AORTIC DISSECTION, AND RELATED PREDICTION DEVICE**

(30) Priority: 08.12.2022 CN 202211569505
(71) Applicant: BEIJING INSTITUTE OF HEART, LUNG AND BLOOD VESSEL DISEASES, Beijing 100029 (CN)
(72) Inventor: DU, Jie, Beijing 100029 (CN); WANG, Yuan, Beijing 100029 (CN); JIANG, Wenxi, Beijing 100029 (CN); WANG, Xue, Beijing 100029 (CN); LI, Fengjuan, Beijing 100029 (CN)
(74) Representative: Frick, Robert
(86) International application number: PCT/CN2023/114561
(87) International publication number: WO 2024/119898

(57) **Abstract**

Use of serum soluble ST2 in preparation of a product for predicting prognosis of a patient with aortic dissection and related prediction device. The levels of soluble ST2 in the serum of a patient with aortic dissection is quantified using ELISA technology, and the serum soluble ST2 can be used as a predictive index for preoperative malperfusion and adverse events during hospitalization of a patient with aortic dissection, and can be used for preparing a product for prediction or auxiliary prediction of prognosis of a patient with aortic dissection. The prognosis of a patient with aortic dissection who has a low serum sST2 level is better than that of a patient with aortic dissection who has a high serum sST2 level. The risk estimation capability for MAEs of a patient with aortic dissection can be improved based on known risk factors, and can be used to accurately stratify patient risks and improve patient survival rate.

## Description

### Technical field

The present invention belongs to the field of diagnosis and specifically to use of serum soluble ST2 for preparation of a product for predicting prognosis of a patient with aortic dissection and related prediction devices.

### Background Art

Aortic Dissection (AD) is a potentially fatal clinical emergency, with an untreated mortality rate of approximately 1% -2% per hour after symptom onset. The European Society of Cardiology clinical guidelines recommend emergency surgery for patients with type A AD (grade I, grade B). However, according to the diagnosis and treatment strategies for acute aortic dissection combined with coronary heart disease, even with surgical treatment, the in-hospital mortality of patients having Stanford type A aortic dissection (AAD) with coronary hypoperfusion or severe coronary artery disorder still exceeds 50%. Therefore, the prognostic information of patients undergoing surgery is crucial for guiding clinical prevention strategies.

In the past few decades, malperfusion has been considered a strong predictor of adverse outcomes in patients with acute type A AD undergoing surgery. Evaluating malperfusion typically requires information from computed tomography and magnetic resonance imaging, but due to their complexity, inconsistency when applied to individuals, and unavailability of important data, they are not routinely used in clinical practice. In addition, there is an increasing amount of research on biomarkers for assessing prognosis of aortic dissection. Although fibrin related markers (D-Dimer), inflammation related markers (C-reactive protein), and cardiovascular biomarkers (NT proBNP or cardiac troponin) have been shown to be associated with clinical outcomes of AD patients, none of them are recommended as prognostic biomarkers AD in clinical guidelines.

ST2 is a receptor for interleukin-33 (IL-33), including soluble ST2 receptor (sST2) and membrane-bound functional receptor (ST2L). Under excessive mechanical load, sST2 acts as a "bait" receptor for IL-33 and is secreted into the circulation. sST2 inhibits the cardioprotective mechanism of the IL33/ST2 axis by competitively binding to IL-33.

### Summary of the invention

The technical problem to be solved by the present invention is how to prepare a product for predicting prognosis of a patient with aortic dissection and/or how to obtain a marker for predicting prognosis of a patient with aortic dissection.

In order to solve the above technical problem, the present invention first provides a use of a system for detecting soluble ST2 (sST2) content in the preparation of a product for predicting or assisting in prediction of prognosis of a patient with aortic dissection, or in the prediction or assisting in prediction of prognosis of a patient with aortic dissection.

In the above use, the system can be a product. The system may include an agent, a kit, and/or an instrument required for detecting the soluble ST2 content.

In the above use, the product may include an agent, a kit, and/or an instrument required for detecting soluble ST2 content. The soluble ST2 content can be serum soluble ST2 content of a patient with aortic dissection to be tested.

In the above use, the agent can include a substance that detects content of the soluble ST2 by enzyme-linked immunosorbent assay, immunofluorescence, radioimmunoassay, immunocoprecipitation, immunoblotting, high performance liquid chromatography, capillary gel electrophoresis, near-infrared spectroscopy, mass spectrometry, immunochemiluminescence, colloidal gold immunoassay, fluorescence immunochromatography, surface plasmon resonance, immuno-PCR or biotin avidin technology.

In the above use, the agent may include soluble ST2 (as a quantitative standard). The agent may also include soluble ST2 (as a quantitative standard) and antibodies that specifically bind to soluble ST2.

In the above use, the kit can be an enzyme-linked immunosorbent assay kit.

In the above use, the prognosis may be the one whether the patient with aortic dissection has preoperative organ malperfusion and/or adverse events during hospitalization.

The preoperative organ malperfusion can be evaluated based on CT images, clinical features, and biomarker levels of patients with aortic dissection. The organ malperfusion may include at least one of the following:
1) coronary artery malperfusion: ischemic electrocardiogram changes, elevated troponin levels, and local wall motion abnormalities on echocardiography;
2) liver malperfusion: elevated aspartate transaminase (>45U/L) and elevated alanine aminotransferase (>60U/L);
3) mesenteric malperfusion: abdominal pain, palpation tenderness (pain symptoms, chief complaint), elevated lactate (>2.25mmol/L);
4) renal malperfusion: creatinine (>111umol/L), blood urea nitrogen (>18mg/dl);
5) neurological malperfusion: transient ischemic attack, limb ischemia (disappearance of pulse, clinical symptoms of limb ischemia, and elevated creatinine kinase levels). The organ malperfusion may refer to the presence of at least one of the above-mentioned organ malperfusion.

The adverse events during hospitalization may include MAEs. The MAEs is defined as a need for rethoracotomy operation and/or an occurrence of postoperative acute heart failure, renal failure, respiratory distress syndrome, neuroischemic injury, sepsis, or death.

In order to solve the above technical problem, the present invention also provides a device for predicting or assisting in predicting the prognosis of a patient with aortic dissection, the device may include a data receiving module and a data processing module; the data receiving module is configured to receive the content of soluble ST2 in the serum of patients with aortic dissection to be tested; the data processing module is used to convert the content of soluble ST2 from the data receiving module into a risk value for the prognosis of the patient with aortic dissection to be tested, and to predict the prognosis of the patient with aortic dissection to be tested based on the risk value.

In the above device, the prognosis may be the one whether the patient with aortic dissection has experienced preoperative organ malperfusion and/or adverse events during hospitalization.

The preoperative organ malperfusion can be evaluated based on CT images, clinical features, and biomarker levels of a patient with aortic dissection. The organ malperfusion may include at least one of the following:
1) coronary artery malperfusion: ischemic electrocardiogram changes, elevated troponin levels, and local wall motion abnormalities on echocardiography;
2) liver malperfusion: elevated aspartate transaminase (>45U/L) and elevated alanine aminotransferase (>60U/L);
3) mesenteric malperfusion: abdominal pain, palpation tenderness (pain symptoms, chief complaint), elevated lactate (>2.25mmol/L);
4) renal malperfusion: creatinine (>111umol/L), blood urea nitrogen (>18mg/dl);
5) neurological malperfusion: transient ischemic attack, limb ischemia (disappearance of pulse, clinical symptoms of limb ischemia, and elevated creatinine kinase levels). The organ malperfusion may refer to the presence of at least one of the above-mentioned organ malperfusion.

The adverse events during hospitalization may include MAEs. The MAEs is defined as a need for rethoracotomy operation and/or an occurrence of postoperative acute heart failure, renal failure, respiratory distress syndrome, neuroischemic injury, sepsis, or death.

The risk value of soluble ST2 (sST2) can be 84.6 ng/mL. A risk of adverse events during hospitalization may be higher in patients with aortic dissection who have serum sST2 content of above 84.6 ng/mL than in patients with aortic dissection who have serum sST2 content of below 84.6 ng/mL. The prognosis of patients with aortic dissection who have serum sST2 content of below 84.6 ng/mL may be better than that of pateints who have serum sST2 content of above 84.6 ng/mL. The patients with aortic dissection can be Chinese.

In order to solve the above technical problem, the present invention also provides a use of serum soluble ST2 as a biomarker in the preparation of a product for detecting prognosis of a patient with aortic dissection, or in the detection of the prognosis of a patient with aortic dissection.

In the above use, the prognosis may be the one whether the patient with aortic dissection has preoperative organ malperfusion and/or adverse events during hospitalization. The product may be an agent, a kit, and/or a device.

In the above use, the agent can include substances that detect the content of the soluble ST2 by enzyme-linked immunosorbent assay, immunofluorescence, radioimmunoassay, immunocoprecipitation, immunoblotting, high performance liquid chromatography, capillary gel electrophoresis, near-infrared spectroscopy, mass spectrometry, immunochemiluminescence, colloidal gold immunoassay, fluorescence immunochromatography, surface plasmon resonance, immuno-PCR or biotin avidin technology.

In the above use, the agent may include soluble ST2 (as a quantitative standard). The agent may also include soluble ST2 (as a quantitative standard) and antibodies that specifically bind to soluble ST2.

In the above use, the kit can be an enzyme-linked immunosorbent assay kit.

In order to solve the above technical problem, the present invention also provides a computer-readable storage medium for predicting or assisting in predicting the prognosis of a patient with aortic dissection, the computer-readable storage medium can enable the computer to run the following steps:
detecting a content of soluble ST2 in the serum of a patient with aortic dissection to be detected, converting the content into a risk value for the prognosis of the patients with aortic dissection to be detected, and predicting the prognosis of the patients with aortic dissection to be detected based on the risk value.

In the above computer-readable storage medium, the prognosis may be the one whether the patient with aortic dissection has preoperative organ malperfusion and/or adverse events during hospitalization.

The preoperative organ malperfusion can be evaluated based on CT images, clinical features, and biomarker levels of the patient with aortic dissection. The organ malperfusion may include at least one of the following:
1) coronary artery malperfusion: ischemic electrocardiogram changes, elevated troponin levels, and local wall motion abnormalities on echocardiography;
2) liver malperfusion: elevated aspartate transaminase (>45U/L) and elevated alanine aminotransferase (>60U/L);
3) mesenteric malperfusion: abdominal pain, palpation tenderness (pain symptoms, chief complaint), elevated lactate (>2.25mmol/L);
4) renal malperfusion: creatinine (>111umol/L), blood urea nitrogen (>18mg/dl);
5) neurological malperfusion: transient ischemic attack, limb ischemia (disappearance of pulse, clinical symptoms of limb ischemia, and elevated creatinine kinase levels). The organ malperfusion may be the presence of at least one of the above-described organ malperfusion.

The adverse events during hospitalization may include MAEs. The MAEs is defined as a need for rethoracotomy operation and/or an occurrence of postoperative acute heart failure, renal failure, respiratory distress syndrome, neuroischemic injury, sepsis, or death.

In order to solve the above technical problem, the present invention also provides a method for predicting the prognosis of a patient with aortic dissection, comprising: detecting a content of soluble ST2 (sST2) in the serum of a patient with aortic dissection to be detected, converting the content into a risk value for the prognosis of the patient with aortic dissection to be detected, and predicting the prognosis of the patients with aortic dissection to be detected based on the risk value.

Specifically, a patient having aortic dissection with serum sST2 content higher than 84.6 ng/mL has a higher or candidate higher risk of developing adverse events during hospitalization than a patient having aortic dissection with serum sST2 content lower than 84.6 ng/mL. A patient having aortic dissection with serum sST2 content below 84.6 ng/mL has better or candidate better prognosis than a patient having aortic dissection with serum sST2 levels above 84.6 ng/mL.

In the above method, the prognosis may be the one whether the patient with aortic dissection has preoperative organ malperfusion and/or adverse events during hospitalization.

The preoperative organ malperfusion can be evaluated based on CT images, clinical features, and biomarker levels of the patient with aortic dissection. The organ malperfusion may include at least one of the following:
1) coronary artery malperfusion: ischemic electrocardiogram changes, elevated troponin levels, and local wall motion abnormalities on echocardiography;
2) liver malperfusion: elevated aspartate transaminase (>45U/L) and elevated alanine aminotransferase (>60U/L);
3) mesenteric malperfusion: abdominal pain, palpation tenderness (pain symptoms, chief complaint), elevated lactate (>2.25mmol/L);
4) renal malperfusion: creatinine (>111umol/L), blood urea nitrogen (>18mg/dl);
5) neurological malperfusion: transient ischemic attack, limb ischemia (disappearance of pulse, clinical symptoms of limb ischemia, and elevated creatinine kinase levels). The organ malperfusion may be the presence of at least one of the above described organ malperfusion.

The adverse events during hospitalization may include MAEs. The MAEs is defined as a need for rethoracotomy operation and/or an occurrence of postoperative acute heart failure, renal failure, respiratory distress syndrome, neuroischemic injury, sepsis, or death.

In the above method, the detecting a content of soluble ST2 (sST2) in the serum of a patient with aortic dissection to be detected can be achieved using an agent, a kit, and/or an instrument required for detecting the soluble ST2 content.

In the above methods the agent can include a substance that detects the content of the soluble ST2 by enzyme-linked immunosorbent assay, immunofluorescence, radioimmunoassay, immunocoprecipitation, immunoblotting, high performance liquid chromatography, capillary gel electrophoresis, near-infrared spectroscopy, mass spectrometry, immunochemiluminescence, colloidal gold immunoassay, fluorescence immunochromatography, surface plasmon resonance, immuno-PCR or biotin avidin technology.

In the above method, the agent may include soluble ST2 (as a quantitative standard). The agent may also include soluble ST2 (as a quantitative standard) and antibodies that specifically bind to soluble ST2.

In the above method, the kit can be an enzyme-linked immunosorbent assay kit.

In order to solve the above technical problem, the present invention also provides a prognostic product for a patient with aortic dissection, comprising an agent, a kit, and/or an instrument required for detecting soluble ST2 content.

In the above product, the agent can include substances that detect the content of the soluble ST2 by enzyme-linked immunosorbent assay, immunofluorescence, radioimmunoassay, immunocoprecipitation, immunoblotting, high performance liquid chromatography, capillary gel electrophoresis, near-infrared spectroscopy, mass spectrometry, immunochemiluminescence, colloidal gold immunoassay, fluorescence immunochromatography, surface plasmon resonance, immuno-PCR or biotin avidin technology.

In the above product, the agent may include soluble ST2 (as a quantitative standard). The agent may also include soluble ST2 (as a quantitative standard) and antibodies that specifically bind to soluble ST2.

In the above product, the kit can be an enzyme-linked immunosorbent assay kit.

The above product can be composed of the agent, the kit, and/or the instrument required for detecting soluble ST2 content, along with other substances used for detecting soluble ST2 content, or can only be the agent, the kit, and/or the instrument required for detecting soluble ST2 content.

In the above product, the soluble ST2 content can be the content of soluble ST2 in the serum.

In the above product, the prognosis may be whether the patient with aortic dissection has preoperative organ malperfusion and/or adverse events during hospitalization.

The patient with aortic dissection described above can be a patient with Stanford type A aortic dissection. The patient described above can be Chinese population.

The above use or method can be non-disease diagnostic use or method, or may be use or method for diagnosis of disease. The above use or method may not have the direct purpose of obtaining disease diagnosis results or health conditions of living human or animal bodies, or may also have the direct purpose of obtaining disease diagnosis results or health conditions of living human or animal bodies.

The above use or method can be use or method for non-disease treatment purposes or for disease treatment purposes. The above use or method may not have the direct purpose of restoring or obtaining health or reducing pain in living human or animal bodies, or may also have the direct purpose of restoring or obtaining health or reducing pain in living human or animal bodies.

The present invention is further described in detail as below in conjunction with specific embodiments. The provided embodiments are only intended to illustrate the present invention and not to limit its scope. The Examples provided below can serve as a guide for those skilled in the art to further improve and do not in any way limit the present invention.

### Brief Description of the Drawings

Figure 1 shows relevant information of patients in discovery cohort and validation cohort. A is the relevant information of patients in discovery cohort; B is the relevant information of the patients in validation cohort.
Figure 2 shows a comparison of serum sST2 levels in patients having aortic dissection combined with/without malperfusion. The vertical axis represents the serum sST2 level, measured in ng/mL.
Figure 3 shows the serum sST2 levels of patients having aortic dissection combined with/without malperfusion in the discovery cohort.
Figure 4 shows the correlation analysis between the serum sST2 levels and the number of organs with malperfusion in patients having aortic dissection combined with malperfusion.
Figure 5 shows a comparison of content of serum sST2 levels of patients between MAES group and Non-MAES group in the discovery cohort.
Figure 6 shows a comparison of serum sST2 levels of patients between MAEs group and Non-MAEs group in the discovery cohort. The vertical axis represents the serum sST2 level, measured in ng/mL.
Figure 7 shows a content comparison of serum sST2 levels of patients between the MAES group and the Non-MAES group in the validation cohort.
Figure 8 shows a comparison of serum sST2 levels of patients between MAEs group and Non-MAEs group in the validation cohort. The vertical axis represents the serum sST2 level, measured in ng/mL.
Figure 9 shows a comparison of ability for counting sST2 levels to assess risk of MAEs using different logistic regression models.
Figure 10 is receiver operating characteristic curves of sST2 distinguishing MAEs from Non-MAEs patients in the validation cohort. The area under the curve is 0.75.

### Detailed description

The experimental methods in the following examples, unless otherwise specified, are all conventional methods. The materials, reagents, instruments, etc. used in the following examples can be obtained from commercial sources unless otherwise specified. The quantitative experiments in the following examples were conducted with three replicates, and the results were averaged.

The relevant definitions in the examples of the present invention are as follows:
Adverse events during hospitalization: major adverse events (MAEs) that occur in patients during hospitalization, which is defined as a need for rethoracotomy operation and/or an occurrence of postoperative acute heart failure, renal failure, respiratory distress syndrome, neuroischemic injury, sepsis, or death.

Organ malperfusion: an evaluation for improper preoperative perfusion based on CT images, clinical features, and biomarker levels. Multiple organ malperfusion is at least one of the following: 1) coronary artery malperfusion: ischemic electrocardiographic changes, elevated troponin levels, and local wall motion abnormalities on echocardiography; 2) liver malperfusion: elevated aspartate transaminase (>45U/L) and elevated alanine aminotransferase (>60U/L); 3) mesenteric malperfusion: abdominal pain, palpation tenderness (pain symptoms, chief complaint), elevated lactate (>2.25mmol/L); 4) renal malperfusion: creatinine (>111umol/L), blood urea nitrogen (>18mg/dl); 5) neurological malperfusion: transient ischemic attack, limb ischemia (disappearance of pulse, clinical symptoms of limb ischemia, and elevated creatinine kinase levels). Organ malperfusion refers to at least presence of one organ malperfusion.

The experimental protocol in the examples of the present invention was approved by Ethics Committee of Beijing Anzhen Hospital affiliated with Capital Medical University, and the study was conducted in accordance with the principles of the Helsinki Declaration. Before enrollment, each patient or legal representative thereof from Beijing Anzhen Hospital affiliated with Capital Medical University signed a written informed consent form.

### Example 1: The expression level of sST2 in the serum of patients with aortic dissection is correlated with preoperative malperfusion.

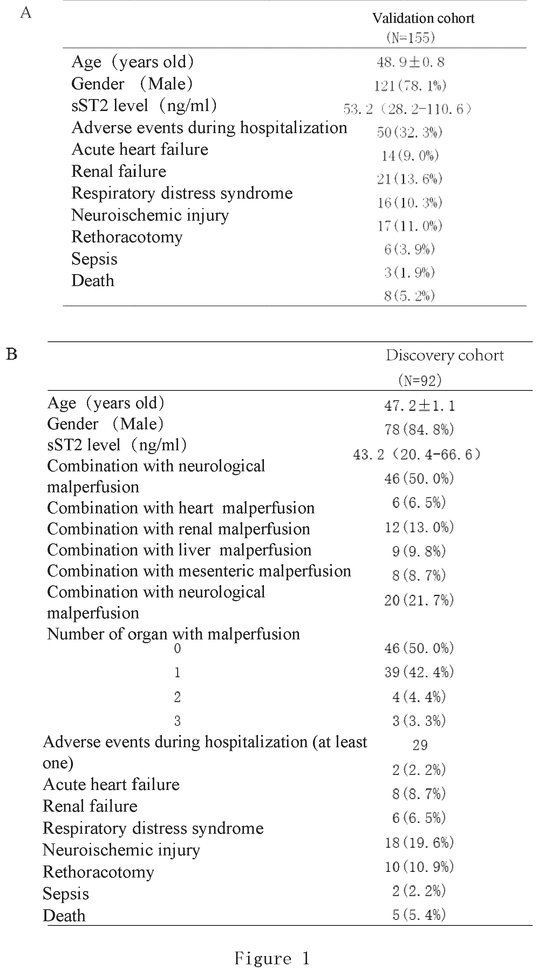

According to the principle of gender and age matching, 46 patients having aortic dissection combined with malperfusion and 46 patients having aortic dissection not combined with malperfusion were selected as a discovery cohort for detecting prognosis of aortic dissection patients. The soluble receptor (sST2) levels of serum interleukin-33 (IL-33) of patients were detected by ELISA, and correlation analysis with preoperative malperfusion was conducted. The malperfusion refers to at least presence of one organ malperfusion. The average age of 92 patients was 47.2 years, with 78 males accounting for 84.8%. The malperfusion and specific MAEs are shown in Figure 1.

### 1. Detection of serum sST2 levels.

Kit: Human ST2/IL-33R DuoSet. Catalog Number: DY523B-05. Company: R&D. Steps of ELISA method for detecting serum sST2:
1) dilute a capture antibody to a working concentration in a PBS solution without carrier protein. Add 100 µL of diluted capture antibody (included in a kit) to each well of a plate, seal the plate, and incubate overnight at room temperature;
2) suck out the solution from each well and wash it with a wash buffer. Repeat this process twice, for a total of three washes. After the final washing, invert the plate and transfer the remaining washing buffer onto a clean tissue;
3) add 300uL of Reagent Diluent (included in the kit) to each well. Incubate the plate at room temperature for at least 1 hour;
4) repeat sucking and washing of step 2;
5) add 100 µ L of sample or standard to each well (included in the kit). Cover with adhesive tape and incubate it at room temperature for 2 hours;
6) repeat the sucking/washing steps in step 2);
7) add 100 µ L of detection antibody (included in the kit) to each well and dilute with reagent diluent. Cover the plate with a new membrane and incubate it at room temperature for 2 hours;
8) repeat the sucking/washing steps in Step 2) of the plate preparation;
9) add 100 µL of Streptavidin HRP working diluent to each well. Cover the plate with a membrane and incubate it at room temperature for 20 minutes. Avoid placing the plate under direct sunlight;
10) repeat step 2) of sucking and washing;
11) add 100 µL of substrate solution to each well (included in the kit). Incubate the plate at room temperature for 20 minutes. Avoid placing the plate under direct sunlight;
12) add 50 µL of termination solution to each well. Gently tap the plate to ensure thorough mixing;
13) set a microplate reader to OD₄₅₀ₙₘ and immediately measure the optical density of each well.

### 2. Analysis of test results.

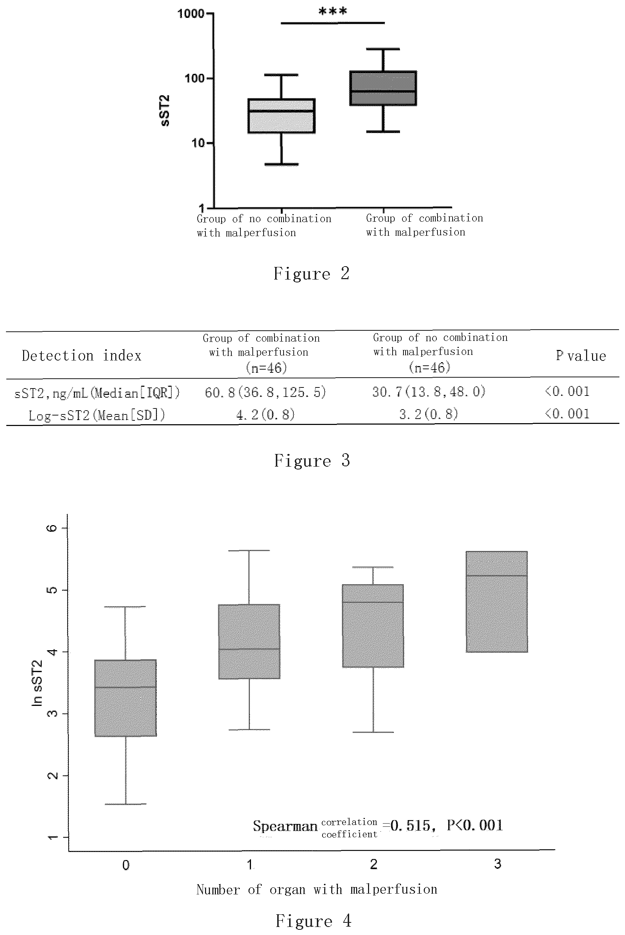

In the discovery cohort, combining with imaging and laboratory testing index, it was determined that there were 46 patients with aortic dissection combined with malperfusion in the discovery cohort. After gender and age matching, 46 patients with aortic dissection not combined with malperfusion were selected. The detection results of sST2 level were shown in Figure 2 and Figure 1. Compared with patients without perfusion in aortic dissection (represented by the group not combined with malperfusion in Figure 2 and Figure 3), patients combined with malperfusion (represented by the group combined with malperfusion in Figure 2 and Figure 3) had significantly increased serum sST2 levels. The results in Figure 3 showed that the median level of sST2 in the group of patients having aortic dissection not combined with malperfusion was 30.7 ng/mL, with the first and third quantiles being 13.8 ng/mL and 48.0 ng/mL, respectively. The median level of sST2 in the group of patients combined with malperfusion was 60.8 ng/mL, with the first and third quantiles being 36.8 ng/mL and 125.5 ng/mL, respectively. The t-test analysis showed that compared with the group of patients not combined with malperfusion, the expression level of sST2 in patients combined with malperfusion was significantly increased (P<0.001). The correlation between sST2 and the number of organ malperfusion was analyzed by Spearman, and the results were shown in Figure 4. The Spearman correlation coefficient was 0.515 (P<0.001), indicating a strong correlation between sST2 and the number of organ with malperfusion, i.e., the more the organs with malperfusion, the higher the level of sST2.

### Example 2: The prognostic evaluation value of sST2 expression level in serum of patients with aortic dissection for in-hospital MAES.

1. Comparison of sST2 expression levels between MAES group and Non-MAES group patients.

Among the 92 patients with aortic dissection included in the discovery cohort, based on occurrence of MAEs (death, heart failure, etc.) events during their hospitalization, the patients with aortic dissection in Example 1 were divided into MAEs group (29 people) and Non-MAEs group (63 people). The serum sST2 levels of the patients were detected using the same ELISA experimental method as in Example 1. Wherein, MAES is an adverse event during hospitalization, defined as a need for re-thoracotomy operation and/or an occurrence of postoperative acute heart failure, renal failure, respiratory distress syndrome, neuroischemic injury, sepsis after surgery, or death.

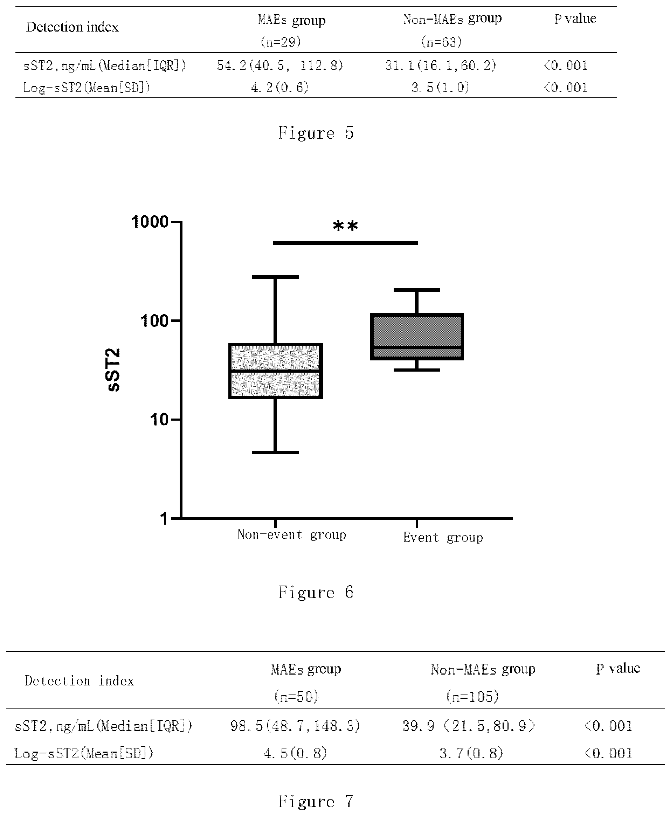

The statistical results showed that:
The comparative data of sST2 content in blood samples statistically analyzed according to grouping were shown in Figure 5 and Figure 6. The median level of sST2 level in the patients of Non-MAEs group was 31.1 ng/mL, with the first and third quantiles being 16.1 ng/mL and 60.2 ng/mL, respectively. The median level of sST2 level in the patients of MAEs group was 54.2 ng/mL, with the first and third quantile being 40.5 ng/mL and 112.8 ng/mL, respectively. Compared with the Non-MAEs, the expression level of sST2 in patients of MAEs was significantly increased (P<0.001).

2. Analysis of the relationship between sST2 levels and MAES through logistic regression models.

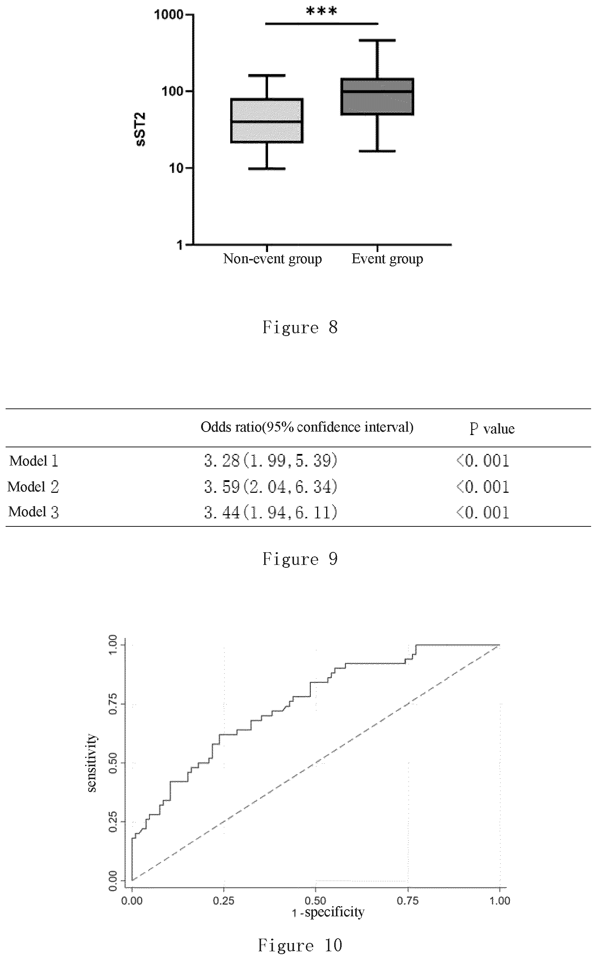

155 patients with aortic dissection (different from the 92 patients in Example 1, they were newly enrolled patients) were selected as validation cohort, with a total of 50 patients who experienced cardiovascular adverse events (death, heart failure, etc.) during hospitalization. The average age of 155 patients was 48.9 years, with 121 males accounting for 78.1%. The patients with aortic dissection in the validation cohort were divided into MAEs group (50 people) and Non-MAEs group (i.e. non MAEs group, 105 people). The serum sST2 levels of patients were detected using the same ELISA experimental method as in Example 1 to verify the concentration difference between the MAEs group patients and Non-MAEs. The results were shown in Figures 7 and 8. The median level of sST2 level in the patients of Non-MAEs group was 39.9 ng/mL, with the first and third quantiles being 21.5 ng/mL and 80.9 ng/mL, respectively. The median level of sST2 level in the patients of MAEs group was 98.5 ng/mL, with the first and third quantiles being 48.7 ng/mL and 148.3 ng/mL, respectively. The serum sST2 levels in the patients of MAEs group (represented by the event group in Figure 8) in the validation cohort were still significantly higher than that in the Non-MAEs group (represented by the non-event group in Figure 8, P<0.001), consistent with the results of the discovery cohort. That is, the risk of adverse events during hospitalization is higher in patients with aortic dissection who have high serum sST2 levels than in patients with aortic dissection who have low serum sST2 levels; the prognosis of the patients with aortic dissection who have low serum sST2 levels is better than that of the patients with aortic dissection who have high serum sST2 levels.

Based on known risk factors of aortic dissection, including age, gender, coronary heart disease, diabetes, hyperlipidemia, hypertension, sudden pain, ECG abnormalities, pulse shortness, renal failure, and hypotension/shock/tamponade, three logistic regression models were established to evaluate the evaluation value of sST2 for MAEs in aortic dissection patients. In Model 1, with sST2 as an independent variable and MAEs as a dependent variable, in the case that no correction for any known risk factors is conducted, risk estimation capacity of sST2 for MAEs was analyzed; in model 2, similarly, with sST2 as an independent variable and MAEs as a dependent variable, the known risk factors of patients' basic characteristics, including gender, age, coronary heart disease, diabetes, hyperlipidemia, and hypertension, were included to assess an independent risk estimation capacity of sST2 for MAEs after correcting the patient's characteristics; in Model 3, based on Model 2, in addition to the known risk factors of patient's basic characteristic, correction for risk factors of patient' onset characteristic (including sudden pain, abnormal electrocardiogram, pulse shortness, renal failure, and hypotension/shock/tamponade) was added to assess the independent risk assessment capacity of sST2 for MAEs after correcting for patient's basic characteristics and onset characteristics.

Three logistic regression models were established, respectively, as follows:
Model 1: no correction for any factors;
Model 2: correction for gender, age, coronary heart disease, diabetes, hyperlipidemia, and hypertension were conducted;
Model 3: correction for age, gender, coronary heart disease, diabetes, hyperlipidemia, hypertension, sudden pain, ECG abnormalities, pulse shortness, renal failure, and hypotension/shock/tamponade were conducted.

The analysis results of different logistic regression models were shown in Figure 9. When no correction for any factors was conducted (Model 1), an odds ratio of sST2 to MAEs risk was 3.28 (95% confidence interval: 1.99-5.39, P<0.001); after correction for gender, age, coronary heart disease, diabetes, hyperlipidemia and hypertension (model 2) was conducted , an odds ratio of sST2 to MAEs risk was 3.59 (95% confidence interval: 2.04-6.34, P<0.001); after correction for gender, age, coronary heart disease, diabetes, hyperlipidemia, hypertension, sudden pain, ECG abnormalities, pulse shortness, renal failure, and hypotension/shock/tamponade factors (model 3) was conducted, an odds ratio of sST2 to MAEs risk was 3.44 (95% confidence interval: 1.94-6.11, P<0.001). The above results indicate that sST2 has an ability to evaluate MAE risk and can still independently predict MAE risk after correcting known risk factors. Finally, using the receiver operating characteristic curve, the critical value (risk value) of sST2 was determined to be 84.6 ng/mL and the area under the curve was 0.75 in the validation cohort (Figure 10), which can effectively distinguish between aortic dissection MAEs and Non-MAEs groups. That is, patients with aortic dissection having serum sST2 content higher than 84.6 ng/mL had a higher risk of adverse events during hospitalization compared to that of patients with aortic dissection having serum sST2 content lower than 84.6 ng/mL; the prognosis of patients with aortic dissection having serum sST2 content below 84.6 ng/mL is better that of patients with aortic dissection having serum sST2 content above 84.6 ng/mL. All the patients in the above study were Chinese population.

The evaluation of MAEs during hospitalization for patients with aortic dissection is currently mainly based on the patient's basic characteristics and onset features in clinical practice. However, despite intervention treatment for patients, the mortality rate of patients with aortic dissection remains high. This is because, besides aortic rupture, the most important cause of death is patient's concomitant organ malperfusion. However, there is currently no effective evaluation index for prognosis of patients, especially for the organ perfusion after onset of the disease. The present invention proposes use of a simple and easily measurable index sST2, which can improve a capacity for predicting and evaluating MAEs risk in patients with aortic dissection based on known risk factors by evaluating the malperfusion of organs in patients, accurately stratifying patient risks, thereby improving survival rate of patient in clinical practice.

### Industrial applications

From the perspective of guiding clinical prevention strategies for patients with aortic dissection, the present invention detected the expression level of sST2 in patients with aortic dissection and found that sST2, as a marker involved in occurrence and development of aortic dissection, may be used to indicate preoperative malperfusion and is independently associated with the occurrence of adverse events during hospitalization in type AAD patients undergoing surgery.

Based on the existing clinical problems above, the present invention studied the relationship between serum sST2 levels and multiple organ malperfusion and prognosis of aortic dissection. 247 Stanford type A aortic dissection patients (AAD) who underwent surgical treatment were selected for quantitation of soluble ST2 levels in their serum using ELISA technology. The statistical results showed that soluble ST2 can be used as a predictive index for preoperative malperfusion in AD patients. Compared with existing clinical detection indexes, sST2 can better and effectively distinguish AAD patients who have experienced adverse events during hospitalization.

The present invention utilizes ELISA technology to quantify the levels of soluble ST2 in the serum of patients with aortic dissection, and finds that serum soluble ST2 can be used as a predictive index for preoperative malperfusion in patients with aortic dissection, and can be used to prepare products for predicting or auxiliarily predicting prognosis of patients with aortic dissection. The prognosis of patients with aortic dissection can be predicted by detecting serum soluble ST2 content. Patients with aortic dissection having low serum sST2 levels have a better prognosis than that of patients with aortic dissection having high serum sST2 levels. The present invention can improve a capability for predicting and evaluating MAEs risk in patients with aortic dissection based on known risk factors, and can be used in clinical practice to accurately stratify patient risks and improve survival rate of patients.

## Claims

1. Use of a system for detecting soluble ST2 content in the preparation of a product for predicting or assisting in predicting prognosis of a patient with aortic dissection, or in the prediction or assisting in prediction of prognosis of a patient with aortic dissection.

2. The use according to claim 1, **characterized in that**: the product comprises an agent, a kit, and/or an instrument required for detecting the soluble ST2 content.

3. The use according to claim 1 or 2, **characterized in that**: the prognosis is whether the patient with aortic dissection has preoperative organ malperfusion and/or adverse events during hospitalization.

4. A device for predicting or assisting in predicting prognosis of a patient with aortic dissection, **characterized in that**: the device comprises a data receiving module and a data processing module; the data receiving module is configured to receive content of soluble ST2 in the serum of a patient with aortic dissection to be tested; the data processing module is used to convert the content of soluble ST2 from the data receiving module into a risk value for prognosis of the patient with aortic dissection to be tested, and predict the prognosis of the patient with aortic dissection to be tested based on the risk value.

5. The device according to claim 4, **characterized in that**: the prognosis is whether the patient with aortic dissection has preoperative organ malperfusion and/or adverse events during hospitalization.

6. Use of serum soluble ST2 as a biomarker in the preparation of a product for detecting prognosis of a patient with aortic dissection, or in the detection of prognosis of a patient with aortic dissection.

7. The use according to claim 6, **characterized in that**: the prognosis is whether the patient with aortic dissection has preoperative organ malperfusion and/or adverse events during hospitalization.

8. The use according to claim 6 or 7, **characterized in that**: the product is an agent, a kit, and/or a device.

9. A computer-readable storage medium for predicting or assisting in predicting prognosis of a patient with aortic dissection, **characterized in that** the computer-readable storage medium enables the computer to run following steps: detecting content of soluble ST2 in the serum of the patient with aortic dissection to be tested, converting the content into a risk value for prognosis of the patient with aortic dissection to be tested, and predicting the prognosis of the patient with aortic dissection to be tested based on the risk value.

10. The computer-readable storage medium according to claim 9, **characterized in that**: the prognosis is whether the patient with aortic dissection has preoperative organ malperfusion and/or adverse events during hospitalization.

11. A method for predicting prognosis of a patient with aortic dissection, comprising: detecting content of soluble ST2 in the serum of the patient with aortic dissection to be tested, converting the content into a risk value for prognosis of the patient with aortic dissection to be tested, and predicting the prognosis of the patient with aortic dissection to be tested based on the risk value.

12. The method according to claim 11, **characterized in that**: the prognosis is whether the patient with aortic dissection has preoperative organ malperfusion and/or adverse events during hospitalization.

13. The method according to claim 11 or 12, **characterized in that**: the detecting the content of soluble ST2 in the serum of the patients with aortic dissection to be tested is accomplished using an agent, a kit, and/or an instrument required for detecting the content of soluble ST2.

14. A prognostic product for a patient with aortic dissection, comprising an agent, a kit, and/or an instrument required for detecting soluble ST2 content.

15. The product according to claim 14, **characterized in that**: the prognosis is whether the patient with aortic dissection has preoperative organ malperfusion and/or adverse events during hospitalization.
